# EUROPÄISCHE PATENTSCHRIFT

(11) **EP 2 758 095 B1**
(45) Veröffentlichungstag und Bekanntmachung des Hinweises auf die Patenterteilung: **02.06.2021**
(21) Anmeldenummer: 12761906.2
(22) Anmeldetag: 12.09.2012
(51) Int. Cl.: A61M 1/36, D03D 1/00, D03D 11/00, G01M 3/16, G01N 27/04, A61B 5/02

(54) **WEBVERFAHREN ZUR HERSTELLUNG EINER VIELZAHL VON FEUCHTIGKEITSSENSOREN FÜR EINE VORRICHTUNG ZUR ÜBERWACHUNG EINES PATIENTENZUGANGS UND ZUR DURCHFÜHURUNG DES WEBVERFAHRENS KONFIGURIERTE WEBVORRICHTUNG**
WEAVING METHOD FOR THE PRODUCTION OF A PLURALITY OF MOISTURE SENSORS, AND WEAVING DEVICE CONFIGURED TO PERFORM SAID WEAVING METHOD
PROCÉDÉ DE TISSAGE POUR FABRIQUER UNE PLURALITÉ DE CAPTEURS D'HUMIDITÉ ET DISPOSITIF DE TISSAGE CONFIGURÉ POUR EXÉCUTER LEDIT PROCÉDÉ DE TISSAGE

(30) Priorität: 21.09.2011 DE 102011113838; 21.09.2011 US 201161537085 P
(43) Veröffentlichungstag der Anmeldung: 30.07.2014
(73) Patentinhaber: Fresenius Medical Care Deutschland GmbH, 61352 Bad Homburg v.d.H. (DE)
(72) Erfinder: HEPPE, John, 66606 St. Wendel (DE)
(74) Vertreter: Oppermann, Frank
(86) Internationale Anmeldenummer: PCT/EP2012/003831
(87) Internationale Veröffentlichungsnummer: WO 2013/041196

(56) Entgegenhaltungen:
- EP-A1- 2 360 301
- EP-A2- 1 997 952
- DE-A1-102006 017 340
- FR-A1- 2 785 626
- JP-A- 54 133 196
- US-A1- 2007 089 800

## Beschreibung

Die Erfindung betrifft ein Webverfahren zur Herstellung einer Vielzahl von Feuchtigkeitssensoren für eine Vorrichtung zur Überwachung eines Zugangs zu einem Patienten, insbesondere zur Überwachung des Gefäßzugangs bei einer extrakorporalen Blutbehandlung, bei der Blut eines Patienten über eine arterielle Schlauchleitung, die eine arterielle Kanüle aufweist, von dem Patienten abgeführt wird, und über eine venöse Schlauchleitung, die eine venöse Punktionskanüle aufweist, dem Patienten zugeführt wird.

Auf dem Gebiet der Medizintechnik sind verschiedene Einrichtungen bekannt, mit denen über eine Schlauchleitung Patienten Flüssigkeit entnommen oder Flüssigkeiten dem Patienten zugeführt werden können. Dabei erfolgt der Zugang zu dem Patienten im Allgemeinen mit einem Katheter zum Einführen in Körperorgane oder einer Kanüle zum Punktieren von Gefäßen. Während der Untersuchung oder Behandlung ist ein ordnungsgemäßer Zugang zu dem Patient sicherzustellen. Daher ist es erforderlich, den Patientenzugang zu überwachen. Einen ordnungsgemäßen Patientenzugang setzen insbesondere die extrakorporalen Blutbehandlungsvorrichtungen voraus, die über einen extrakorporalen Blutkreislauf verfügen.

Es sind Vorrichtungen zur Überwachung eines Gefäßzugangs bekannt, die über einen Feuchtigkeitssensor verfügen, um an der Punktionsstelle austretendes Blut detektieren zu können. Die bekannten Feuchtigkeitssensoren sind als Pad ausgebildet, das auf die Punktionsstelle aufgelegt wird. Die WO 2006/008866 A1, US 2005/0038325 A1 und US 6 445 304 B1 beschreiben Feuchtigkeitssensoren, die aus einem saugfähigen Material bestehen, in das eine elektrisch leitende Struktur eingebettet ist. Die Detektion von Feuchtigkeit beruht auf der Messung des elektrischen Widerstands zwischen den Leiterbahnen.

Aus der WO 2009/075592 A2 ist ein Feuchtigkeitssensor in Form eines Gewebestreifen bekannt, auf dem oder in dem zwei parallele Leiterbahnen vorgesehen sind, zwischen denen der elektrische Widerstand gemessen wird. Die beiden Leiterbahnen werden durch leitfähige Garne gebildet, die nur in Längsrichtung des Gewebestreifens verlaufen. Elektrische Kontaktstellen zwischen sich kreuzenden Leiterbahnen sind nicht vorgesehen.

Die JP 54 133196 A beschreibt einen Feuchtigkeitssensor zur Überwachung eines heiße Flüssigkeiten führenden Leitungssystems, welcher mit einem Verfahren hergestellt werden soll, bei dem auf einer Materialbahn mehrere Sensoren in einer Reihe hintereinander angeordnet werden. Die JP 54 133196 A schlägt vor, leitfähige Drähte in ein Gewebe aus hitzeresistenten Fasern einzuweben. Das textile Flächengebilde besteht aus nicht-leitfähigen Kettfäden und Schussfäden und zusätzlichen leitfähigen Drähten, die sich parallel zu den Kettfäden erstrecken.

Aus der DE 10 2006 017340 A1 ist ein elektrisch leitfähiges Garn bekannt, das für die Herstellung von Feuchtigkeitssensoren Verwendung finden und auf Näh-/Stickmaschinen bzw. Strickmaschinen oder Webstühlen verarbeitet kann. Das Garn soll als Klett- und Schussfaden in ein Gewebe eingearbeitet werden.

Die EP 1 997 952 A2 beschreibt eine sogenannte gewebte Interdigitalstruktur, die als textiler Feuchtesensor zum Einsatz kommen kann. Es wird eine Interdigitalstruktur mit einem ersten und zweiten Elektrodenkamm beschrieben, zwischen denen nicht leitfähige Kett- und Schussfäden eine abstandshaltende Gewebestruktur ausbilden. Zur Kontaktierung der Kammelektroden dienen metallisierte Fäden.

Gewebestrukturen, die beispielsweise als EKG-Elektroden Verwendung finden sollen, sind aus der US 2007/0089800 A1 bekannt. Die bekannten textilen Flächengebilde weisen leitfähige und nicht leitfähige Fäden auf.

Der Erfindung liegt die Aufgabe zugrunde, ein Webverfahren anzugeben, mit dem Feuchtigkeitssensoren für eine Vorrichtung zur Überwachung eines Patientenzugangs in großen Stückzahlen kostengünstig hergestellt werden können.

Die Lösung dieser Aufgabe erfolgt erfindungsgemäß mit den Merkmalen des Patentanspruchs 1.

Das erfindungsgemäße Verfahren zur Herstellung einer Vielzahl von Feuchtigkeitssensoren zeichnet sich dadurch aus, dass die Feuchtigkeitssensoren gewebt werden. Beim Weben der Feuchtigkeitssensoren werden nicht-leitfähige Kettfäden und nicht-leitfähige Schussfäden sowie leitfähige Kettfäden und leitfähige Schussfäden in dem textilen Flächengebilde derart angeordnet, dass räumlich abgrenzbare Strukturen aus elektrischen Leiterbahnen geschaffen werden. Die Leiterbahnstrukturen können dabei durch räumliche Trennung bzw. Kontaktierung der leitfähigen Kett- oder Schussfäden erzeugt werden.

Um die Feuchtigkeitssensoren in großen Stückzahlen kostengünstig herstellen zu können, werden auf einer gemeinsamen Gewebebahn mit zunehmenden Webfortschritt wiederholt jeweils eine Mehrzahl von einzelnen Feuchtigkeitssensoren quer zur Produktionsrichtung nebeneinander gewebt, so dass die Breite der Gewebebahn durch möglichst viele Feuchtigkeitssensoren bestmöglich belegt wird. Nach dem Weben und gegebenenfalls weiteren Bearbeitungsschritten werden die einzelnen Feuchtigkeitssensoren voneinander separiert.

Da in einem Arbeitsgang eine Vielzahl von Feuchtigkeitssensoren hergestellt werden kann, ist die Herstellung von Feuchtigkeitssensoren mit relativ geringen Produktionskosten verbunden. Der Produktionsprozess kann mit einem hohen Automatisierungsgrad erfolgen.

Die räumliche Anordnung der Feuchtigkeitssensoren erfolgt verschachtelt. Dadurch ist es möglich, eine besonders große Anzahl von Feuchtigkeitssensoren in Zeilen und Spalten mit einem besonders geringen Abstand zueinander zu weben, sodass in einem Arbeitsgang eine besonders große Anzahl von Feuchtigkeitssensoren hergestellt werden kann.

Für das erfindungsgemäße Webverfahren ist grundsätzlich unerheblich, wie die Leiterbahnstrukturen aus leitfähigen Kett- und/oder Schussfäden beschaffen sind. Auch ist für das erfindungsgemäße Verfahren grundsätzlich unerheblich, wie die räumlich abgrenzbaren Strukturen aus elektrischen Leiterbahnen auf der gemeinsamen Gewebebahn angeordnet sind.

In der Praxis hat sich jedoch gezeigt, dass die Anordnung der Feuchtigkeitssensoren mit den räumlich abgrenzbaren Leiterbahnstrukturen insofern von Bedeutung ist, als dass eine besondere Anordnung der Feuchtigkeitssensoren und der Leiterbahnstrukturen eine besonders effiziente Herstellung der Feuchtigkeitssensoren erlaubt.

Vorteilhafte Ausführungsformen der Erfindung, die Gegenstand der Unteransprüche sind, beziehen sich auf die besondere Anordnung der Feuchtigkeitssensoren mit den Leiterbahnstrukturen auf der Gewebebahn.

Darüber hinaus ist für eine besonders effiziente Herstellung der Feuchtigkeitssensoren von Bedeutung, dass die einzelnen Feuchtigkeitssensoren ohne Zerstörung der Leiterbahnstrukturen voneinander getrennt werden können.

Vorteilhafte Ausführungsformen des erfindungsgemäßen Verfahrens, die eine exakte Separierung der einzelnen Feuchtigkeitssensoren ermöglichen, sind ebenfalls Gegenstand der Unteransprüche.

Die Erfindung betrifft auch eine Vorrichtung zum Weben, mit der das erfindungsgemäße Webverfahren durchführbar ist.

Eine bevorzugte Ausführungsform des erfindungsgemäßen Verfahrens sieht vor, dass zwischen einer vorausgehenden Gruppe von Feuchtigkeitssensoren, die quer zur Produktionsrichtung nebeneinander gewebt werden, und einer nachfolgenden Gruppe von Feuchtigkeitssensoren, die quer zur Produktionsrichtung nebeneinander gewebt werden, ein quer zur Produktionsrichtung verlaufender Ausgleichstreifen gewebt wird.

In den Bereichen der Gewebebahn mit den Feuchtigkeitssensoren kommen lokal unterschiedliche Bindungsarten beim Weben zum Einsatz. Dadurch kommt es zu lokal unterschiedlichen Längenänderungen der Fäden. Diese Längenänderungen verursachen wiederum Spannungen im Gewebe.

Mit den Ausgleichsstreifen kann das Auftreten von Spannungen im Gewebe verhindert werden. Unter einem Ausgleichsstreifen wird ein Bereich auf der Gewebebahn verstanden, der nur eine gleichbleibende Bindungsart aufweist, beispielsweise eine Atlasbindung oder eine Leinwandbindung. Mechanische Spannungen zwischen den Fäden können sich in den Ausgleichstreifen ausgleichen bzw. angleichen. Dadurch ist es möglich, Feuchtigkeitssensoren in dem Gewebe in großer Dichte anzuordnen, ohne dass die Gefahr besteht, dass die exakte Ausrichtung der Feuchtigkeitssensoren mit den Leiterbahnen zueinander verloren geht, die für die Produktion der Sensoren von großer Bedeutung ist.

Die sich kreuzenden leitfähigen Kettfäden und Schussfäden werden zur Bildung der Leiterbahnstrukturen vorzugsweise derart angeordnet, dass die Kett- und Schussfäden an einzelnen Kreuzungspunkten kontaktieren. Vorzugsweise werden die Leiterbahnstrukturen der einzelnen Feuchtigkeitssensoren aus Gruppen von leitfähigen Kettfäden, die jeweils eine Mehrzahl von in einer ersten Richtung verlaufenden leitfähigen Kettfäden umfassen, und Gruppen von leitfähigen Schussfäden, die jeweils eine Mehrzahl von in einer zu der ersten Richtung orthogonalen Richtung verlaufenden leitfähigen Schussfäden umfassen, gebildet. Dabei werden die Gruppen von Kettfäden im gleichen Abstand zueinander angeordnet und/oder die Gruppen von Schussfäden im gleichen Abstand zueinander angeordnet. Dadurch ergibt sich ein weitgehend symmetrischer Aufbau, der aber eine versetzte oder verschachtelte Anordnung der Feuchtigkeitssensoren noch zulässt, wenn die Feuchtigkeitssensoren eine der geschaffenen Struktur entsprechende Form aufweisen.

Die Feuchtigkeitssensoren weisen jeweils einen inneren Bereich mit zwei Schenkeln auf, die einen zentralen Ausschnitt seitlich umschließen. Eine bevorzugte Ausführungsform sieht vor, dass die Feuchtigkeitssensoren jeweils Laschen aufweisen, an der die Leiterbahnen Anschlusskontakte bilden. Die Feuchtigkeitssensoren sind derart zueinander versetzt angeordnet, dass jeweils ein Schenkel eines Feuchtigkeitssensors in einen Ausschnitt eines anderen Feuchtigkeitssensors greift. Der seitliche Versatz der Feuchtigkeitssensoren entspricht dabei dem Abstand der einzelnen Gruppen von Kett- oder Schussfäden, mit denen die Leiterbahnen auf dem mittleren Bereich und den beiden Schenkeln der Feuchtigkeitssensoren gebildet werden.

Bei der verschachtelten Anordnung der Feuchtigkeitssensoren ist von Vorteil, wenn die Sensoren derart ausgebildet sind, dass jeweils nebeneinander angeordnete Sensoren zumindest über einen Teilbereich eine gemeinsame Außenkontur aufweisen. Vorzugsweise können die Feuchtigkeitssensoren derart zueinander versetzt angeordnet werden, dass jeweils ein Schenkel eines Sensors in einen Ausschnitt eines anderen Sensors greift, wobei die verschachtelten Feuchtigkeitssensoren im Bereich der Ausnehmung bzw. im Bereich des Schenkels eine gemeinsame Außenkontur haben. Dadurch entsteht eine lückenlos verschachtelte Anordnung von Feuchtigkeitssensoren, die sich durch eine optimierte Schnittkantenlänge auszeichnet. Die minimierte Schnittkantenlänge führt zu einer Zeitersparnis beim Schneiden, insbesondere Laserschneiden, und einem verringertem Material verschnitt, wobei mit nur einem Schnitt in dem betreffenden Bereich zwei Sensoren voneinander getrennt werden können.

Bei der Ausführungsfonn der Feuchtigkeitssensoren mit dem mittleren Bereich und den beiden Schenkeln werden die Leiterbahnstrukturen vorzugsweise jeweils von drei Gruppen von Schussfäden und zwei Gruppen von Kettfäden gebildet. Dabei umfassen die Gruppen von Schussfäden vorzugsweise die gleiche Anzahl von Schussfäden, während die Gruppen von Kettfäden eine unterschiedliche Anzahl von Kettfäden umfasst. Der symmetrische Aufbau der bevorzugten Ausführungsform der Feuchtigkeitssensoren lässt zu, dass die Gruppen von Schussfäden im gleichen Abstand zueinander angeordnet werden können. Anstelle der Schussfäden können aber auch Kettfäden vorgesehen sein oder umgekehrt.

Quer zur Produktionsrichtung verlaufende Markierungselemente und/oder kreuzförmige Markierungselemente, die zwischen den Feuchtigkeitssensoren angeordnet werden, dienen vorzugsweise dazu, die Positionen der Feuchtigkeitssensoren auf der Gewebebahn zu kennzeichnen. Eine genaue Kennzeichnung und Erkennung der Position der Feuchtigkeitssensoren ist nicht nur beim Separieren der Feuchtigkeitssensoren notwendig, sondern auch dann erforderlich, wenn auf die Gewebebahn eine weitere Lage in exakter Ausrichtung zu den Feuchtigkeitssensoren bzw. Leiterbahnstrukturen aufgebracht werden soll. In der Praxis hat sich gezeigt, dass erst mit dem Weben von Markierungselementen die Produktion der Sensoren in großen Stückzahlen auf einer gemeinsamen Gewebebahn möglich ist.

Bei einer bevorzugten Ausführungsform dient die auf die Gewebebahn aufzubringende Lage dazu, eine Trennschicht und/oder eine Klebeschicht zu schaffen. Mit der Trennschicht kann eine Barriere gegen Durchfeuchtung des Gewebes geschaffen werden, während der Feuchtigkeitssensor mit der Klebeschicht auf der Haut des Patienten fixiert werden kann. Bei der Lage handelt es sich vorzugsweise um eine doppelseitige Klebefolie, die an einer Seite mit einer Abziehfolie (Liner) versehen ist.

Die Markierungselemente werden vorzugsweise durch leitfähige Kett- und/oder Schussfäden gebildet, die sich in der Farbe von den nicht-leitfähigen Kett- bzw. Schussfäden unterscheiden. Vorzugsweise werden die leitfähigen Kett- und/oder Schussfäden, die die Markierungselemente bilden, an die Oberfläche des Gewebes gelegt, so dass sie besser sichtbar sind. Wenn leitfähige Kett- und/oder Schussfäden an der zu markierenden Stelle nicht vorhanden sein sollten, können die Markierungselemente auch kontrastreiche Fäden bilden, die nicht leitfähig sind.

Die sich kreuzenden leitfähigen Kett- und Schussfäden oder kontrastreichen nicht-leitfähigen Fäden erlauben auch die Schaffung von Markierungselementen, die als Kontrollpunkte dienen können.

Die Markierungselemente oder Kontrollpunkte werden vorzugsweise in dem Bereich der Gewebebahn angeordnet, in denen sich die Feuchtigkeitssensoren nicht befinden. Die Markierungselemente können sich aber auch in den Feuchtigkeitssensoren befinden oder durch die Feuchtigkeitssensoren erstrecken, wenn die Leiterbahnstrukturen dadurch nicht zerstört werden.

Im Folgenden wird ein Ausfuhrungsbeispiel einer Blutbehandlungsvorrichtung, welche aber nicht Gegenstand der vorliegenden Erfindung ist und über eine Vorrichtung zur Überwachung des arteriellen und venösen Gefäßzugangs verfügt, sowie ein Ausführungsbeispiel des Feuchtigkeitssensors, der bei der Vorrichtung zur Überwachung des Patientenzugangs Verwendung findet, naher erläutert. Darüber hinaus wird das erfindungsgemäße Verfahren zur Herstellung der Feuchtigkeitssensoren für die Vorrichtung zur Überwachung des Patientenzugangs im Einzelnen beschrieben.

Es zeigen:
- Fig. 1: die wesentlichen Komponenten einer Blutbehandlungsvorrichtung, die über eine Vorrichtung zur Überwachung des arteriellen und venösen Gefäßzugangs verfugt,
- Fig. 2: ein Schnitt durch das Gewebe eines erfindungsgemäßen Feuchtigkeitssensors,
- Fig. 3: ein Ausführungsbeispiel des erfindungsgemäßen Feuchtigkeitssensors in schematischer Darstellung,
- Fig. 4: eine Matrix zur Veranschaulichung der Kreuzungspunkte der Kett- und Schussfäden der Vorrichtung zum Detektieren von Feuchtigkeit von Fig. 3,
- Fig. 5: eine Darstellung zur Veranschaulichung von verschiedenen Schnitten durch die Vorrichtung von Fig. 3,
- Fig. 6 A - Fig. 6 E: eine Darstellung zur Veranschaulichung der Verknüpfungen zwischen Kett- und Schussfäden der Vorrichtung von Fig. 3 in den Schnittebenen von Fig. 5,
- Fig. 7: ein Ausführungsbeispiel der Lasche des Feuchtigkeitssensors,
- Fig. 8: ein weiteres Ausführungsbeispiel der Lasche des Feuchtigkeitssensors,
- Fig. 9: eine stark vereinfachte schematische Darstellung der Verfahrensschritte zur Herstellung des Feuchtigkeitssensors nach dem erfindungsgemäßen Verfahren,
- Fig. 10: die Gewebebahn mit den Feuchtigkeitssensoren, wobei zwischen den Feuchtigkeitssensoren ein Ausgleichstreifen angeordnet ist,
- Fig. 11: eine Darstellung zur Veranschaulichung der kleberfreien Zonen der mit der Gewebebahn zu kaschierenden Klebefolie,
- Fig. 12: eine Darstellung zur Veranschaulichung der Vermaßung der mit der Gewebebahn zu kaschierenden Klebefolie, die mit einer Abziehfolie (Liner) versehen ist,
- Fig. 13: eine Darstellung zur Veranschaulichung der Anordnung von Markierungselementen zur Positionserkennung der Feuchtigkeitssensoren auf der Gewebebahn,
- Fig. 14: eine Darstellung zur Veranschaulichung von Kontrollpunkten zur Überprüfung der Feuchtigkeitssensoren nach dem Separieren und
- Fig. 15: den Feuchtigkeitssensor mit der Anschlusslasche in der Rückansicht.

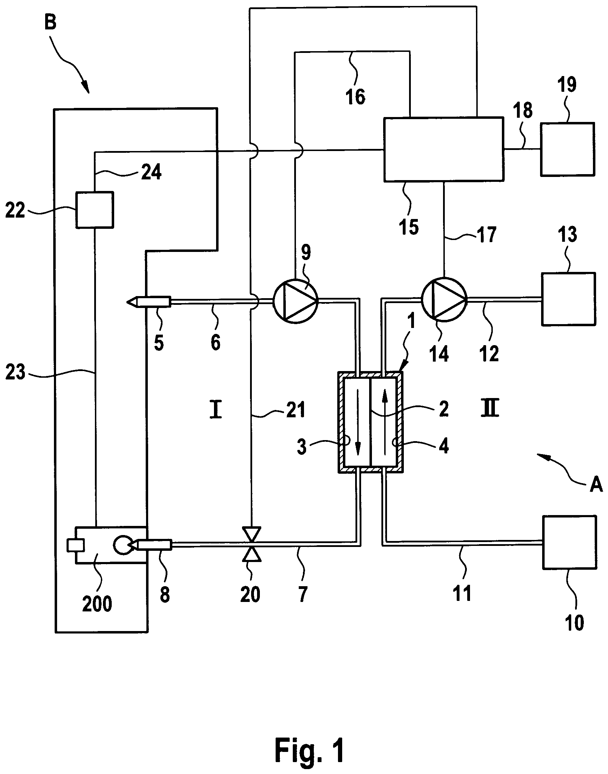

Fig. 1 zeigt die wesentlichen Komponenten einer Blutbehandlungsvorrichtung, insbesondere Hämodialysevorrichtung A, die über eine Vorrichtung B zur Überwachung des arteriellen und venösen Gefäßzugangs verfügt. Bei dem vorliegenden Ausführungsbeispiel ist die Überwachungsvorrichtung B Bestandteil der Hämodialysevorrichtung A. Zunächst wird die Dialysevorrichtung unter Bezugnahme auf Fig. 1 beschrieben.

Die Hämodialysevorrichtung A weist einen Dialysator 1 auf, der durch eine semipermeable Membran 2 in eine Blutkammer 3 und eine Dialysierflüssigkeitskammer 4 unterteilt ist. An der Fistel oder dem Shunt des Patienten ist mittels einer arteriellen Punktionskanüle 5 eine arterielle Schlauchleitung 6 angeschlossen, die zu dem Einlass der Blutkammer des Dialysators führt. Von dem Auslass der Blutkammer 3 des Dialysators 1 geht eine venöse Schlauchleitung 7 ab, die mittels einer venösen Punktionskanüle 8 an der Fistel oder dem Shunt des Patienten angeschlossen ist. In die arterielle Schlauchleitung 6 ist in eine Blutpumpe 9 geschaltet, die das Blut im extrakorporalen Blutkreislauf I fördert. Der Dialysierflüssigkeitskreislauf II der Dialysevorrichtung A umfasst eine Dialyseflüssigkeitsquelle 10, an der eine Dialysierflüssigkeitszuführleitung 11 angeschlossen ist, die zu dem Einlass der Dialysierflüssigkeitskammer 4 des Dialysators führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine Dialysierflüssigkeitspumpe 14 geschaltet.

Die Steuerung der Dialysevorrichtung übernimmt eine zentrale Steuereinheit 15, die über Steuerleitungen 16, 17 die Blut- und Dialysierflüssigkeitspumpe 9, 14 ansteuert. Die zentrale Steuereinheit 15 ist über eine Datenleitung 18 mit einer Alarmeinheit 19 verbunden, die bei einem Störfall einen optischen und/oder akustischen und/oder taktilen Alarm gibt. Stromab der Blutkammer 3 des Dialysators 1 befindet sich an der venösen Schlauchleitung 7 eine elektromagnetisch betätigbare Schlauchklemme 20, die über eine weitere Steuerleitung 21 mit der zentralen Steuereinheit 15 verbunden ist.

Die Überwachungsvorrichtung B dient bei dem vorliegenden Ausführungsbeispiel zur Überwachung des venösen Gefäßzugangs. Die Überwachungsvorrichtung B verfügt über einen Feuchtigkeitssensor 200, der an der Punktionsstelle angeordnet wird. Der Feuchtigkeitssensor 200 ist in Fig. 1 nur schematisch dargestellt. Darüber hinaus verfügt die Überwachungsvorrichtung über eine Auswerteinheit 22, die über eine Verbindungsleitung 23 mit dem Feuchtigkeitssensor 200 elektrisch verbunden ist.

Über eine Datenleitung 24 ist die Auswerteinheit 22 mit der zentralen Steuereinheit 15 der Dialysevorrichtung A verbunden. Für den Fall, dass Blut aus der venösen Kanüle und/oder der Punktionsstelle austritt und den Feuchtigkeitssensor befeuchtet, erzeugt die Auswerteinheit 22 ein Steuersignal, das die zentrale Steuereinheit 15 empfängt, die einen Eingriff in die Blutbehandlung vornimmt. Die Steuereinheit 15 stoppt die Blutpumpe 9 und schließt die Schlauchklemme 20. Darüber hinaus erzeugt die Steuereinheit ein Alarmsignal, sodass die Alarmeinheit 19 einen akustischen und/oder optischen und/oder taktilen Alarm gibt.

Nachfolgend wird ein erstes Ausführungsbeispiel des auf die Haut des Patienten an der Punktionsstelle aufzulegenden Feuchtigkeitssensors 200 beschrieben. Der Feuchtigkeitssensor 200 ist als ein auf die Haut des Patienten aufzulegendes Pad aus einem textilen Flächengebilde (Gewebe) ausgebildet. Bei dem ersten Ausführungsbeispiel ist das textile Flächengebilde 100 ein mehrlagiges Gewebe.

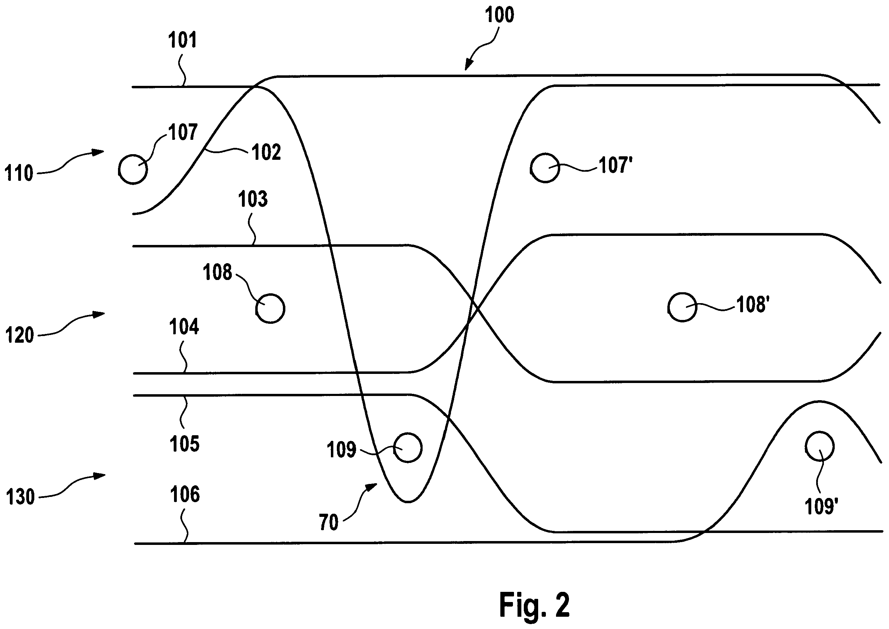

Fig. 2 zeigt einen Kettschnitt durch das mehrlagige Gewebe 100. In Fig. 2 sind die von links nach rechts verlaufenden Kettfäden dargestellt. Der Kettschnitt zeigt insgesamt 6 Kettfäden 101 - 106. Die Anzahl der Lagen des Gewebes ist nach der Anzahl der Ebenen 110, 120, 130 definiert, in der die Schussfäden, 107, 108, 109; 107', 108', 109' liegen. Die in den drei Ebenen 110, 120, 130 im Wesentlichen rechtwinklig zu den Kettfäden liegenden Schussfäden 107, 108, 109; 107', 108', 109' sind durch Kreise gekennzeichnet. Die Herstellung eines dreilagigen Gewebes ist dem Fachmann bekannt. Beim Weben liegen die Schussfäden 107, 108, 109; 107', 108', 109' auf drei Ebenen 100, 110, 120. Die Kettfäden 101- 106 werden auf drei Ebenen zugeführt. Aus den drei Kettfädenebenen heraus kann jeder einzelne Kettfaden jeweils angehoben oder abgesenkt werden, um das Durchschießen eines Schussfadens zu ermöglichen. Bei dem mehrlagigen Gewebe werden in der Produktion aus ursprünglich 60 Fäden/cm auf einer Ebene 20 Fäden in einer oberen Ebene zugeführt, 20 Fäden in einer mittleren Ebene zugeführt und 20 Fäden in einer unteren Ebene zugeführt. Die Anzahl von 60 Fäden/cm stellt ein übliches Beispiel dar, kann jedoch auch davon abweichen.

Die Schussfäden 107, 108, 109; 107', 108', 109' müssen beim Webprozess nicht zwingend in übereinander liegenden Ebenen zugeführt werden, sondern die Lage eines Schussfadens in einer Ebene kann sich im Webprozess auch durch die angehobenen oder abgesenkten Kettfäden ergeben, die den Schussfaden zwangsläufig in eine definierte Ebene ziehen. Die Ebenen sind stets als "gedachte" Lagen zu verstehen, die nicht "plan" sein müssen.

Das Mehrlagengewebe besteht aus elektrisch leitfähigen und elektrisch nicht leitfähigen Kett- und Schussfäden (Monofilamente, Carbonfasern, versilbertes Polyamidgarn). Die elektrisch leitfähigen und elektrisch nicht leitfähigen Kett- und Schussfäden sind derart angeordnet, dass das Gewebe eine der Haut des Patienten zugewandte untere Lage, eine mittlere Lage und eine der Haut des Patienten abgewandte obere Lage aufweist. Die Aufteilung des Gewebes in mehrere Lagen dient aber nur dem besseren Verständnis des Gewebeaufbaus, da sich in der Praxis die Lagen nicht genau voneinander trennen lassen.

Eine Struktur von elektrischen Leiterbahnen wird in der mittleren und oberen Ebene des Gewebes dadurch gebildet, dass die elektrisch leitfähigen Kett- und Schussfäden an den Kreuzungspunkten derart angeordnet werden, dass sie entweder elektrisch leitend miteinander verbunden sind oder elektrisch voneinander isoliert sind. Eine Kontaktstelle zwischen elektrisch leitfähigen Kett- und Schussfäden wird mittels eines partiellen Ebenenwechsels des Kettfadens während des Webprozesses erzielt, wie aus Fig. 2 ersichtlich ist.

Fig. 2 zeigt die in den drei Ebenen 110, 120, 130 liegenden Schussfäden 107, 108, 109; 107', 108', 109'. Durch den partiellen Wechsel des elektrisch leitfähigen Kettfadens 102 beispielsweise von der oberen Ebene 110 in die untere Ebene 130 wird eine elektrische Verbindung zwischen diesem Kettfaden 101 und demjenigen elektrisch leitenden Schussfaden 109 in der unteren Ebene hergestellt, der den Kettfaden 102 kreuzt. Ohne den partiellen Wechsel der Ebenen sind elektrisch leitfähige Kett- und Schussfäden voneinander isoliert. Beispielsweise ist der elektrisch leitfähige Kettfaden 102 nicht elektrisch mit dem elektrisch leitfähigen Schussfaden 109 verbunden, da der Kettfaden 102 im Bereich des Schussfadens 109 die Ebene partiell nicht wechselt.

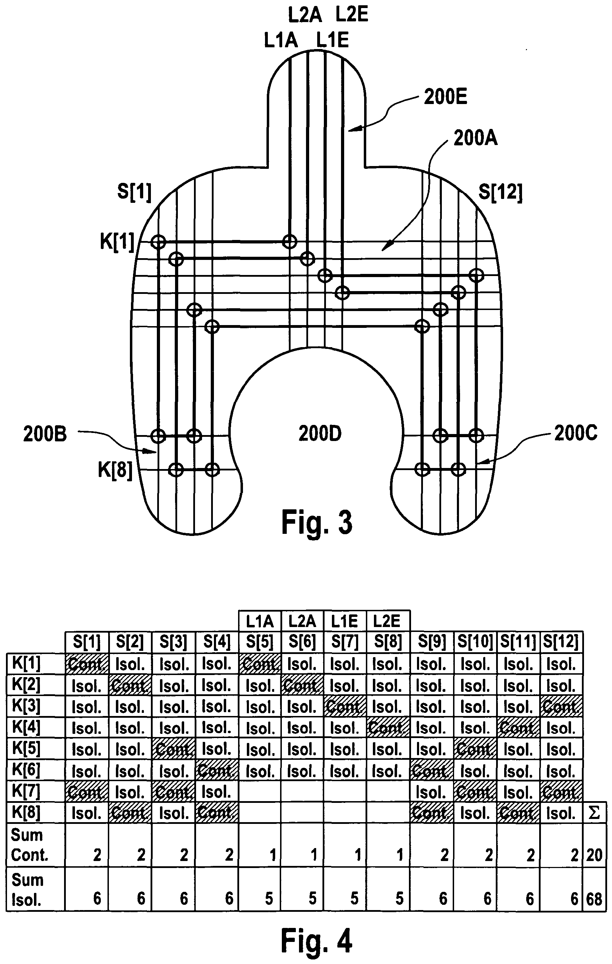

Fig. 3 zeigt ein Ausführungsbeispiel des erfindungsgemäßen Feuchtigkeitssensors in schematischer Darstellung. Der Feuchtigkeitssensor weist einen mittleren Bereich 200A mit zwei Schenkeln 200B, 200C auf, die einen halbkreisförmigen Ausschnitt 200D seitlich umschließen. An dem mittleren Bereich ist eine Lasche 200E angeformt, die beiden Schenkeln gegenüberliegt.

Die eine Struktur aus elektrischen Leiterbahnen bildenden elektrisch leitfähigen Kett- und Schussfäden werden durch horizontale und vertikale dünne Linien gekennzeichnet. Die Schussfäden S verlaufen in vertikaler und die Kettfäden K in horizontaler Richtung. Die Leiterbahnstruktur wird von acht Kettfäden K [1] bis K [8] und zwölf Schussfäden S [1] bis S [12] gebildet, die an den Kreuzungspunkten derart angeordnet sind, dass sie entweder elektrisch leitend verbunden sind oder elektrisch voneinander isoliert sind.

Fig. 4 zeigt eine Matrix zur Veranschaulichung der 88 Kreuzungspunkte der 8 Kettfäden K [1] bis K [8] und 12 Schussfäden S [1] bis S [12]. Die Schnittpunkte zweier leitfähiger Fäden, die einen Kontakt erzeugen, sind in der Matrix mit "Cont." bezeichnet, während die Schnittpunkte zweier leitfähiger Fäden, die eine Isolationsstelle bilden, mit "Isol." bezeichnet sind. Es ergibt sich eine elektrisch leitende Struktur, die zwei Leiterbahnen aufweist, die jeweils eine nicht redundant ausgeführte Leiterschleife bilden.
In Fig. 3 sind die elektrischen Kontaktstellen an den Kreuzungspunkten zwischen den elektrisch leitfähigen Kett- und Schussfäden K [i], S [i] als Kreise dargestellt. Die erste Leiterbahn L1A-L1E verläuft von der Lasche 200E über den mittleren Bereich 200A zu dem linken Schenkel 200B und von dem linken Schenkel über den mittleren Bereich zu dem rechten Schenkel 200C und von dem rechten Schenkel über den mittleren Bereich zurück zu der Lasche des Pads. Der Anfang der jeweiligen Leiterbahn ist mit "A" und das Ende der Leiterbahn ist mit "E" bezeichnet. Die beiden Enden L1A, L1E der ersten Leiterbahn L1A-L1E bilden die beiden Anschlusskontakte. Die zweite Leiterbahn L2A-L2E verläuft von der Lasche 200E über den mittleren Bereich 200A zu dem linken Schenkel 200B und von dem linken Schenkel über den mittleren Bereich zu dem rechten Schenkel 200C und von dem rechten Schenkel über den mittleren Bereich zu der Lasche des Pad 40. Die beiden Enden L2A, L2E der zweiten Leiterbahn L2A-L2E bilden das zweite Paar von Anschlusskontakten. An der Lasche 200E sind die Anschlusskontakte so angeordnet, dass die Anschlusskontakte L2A und L1E zwischen den Anschlusskontakten L1A und L2E liegen.

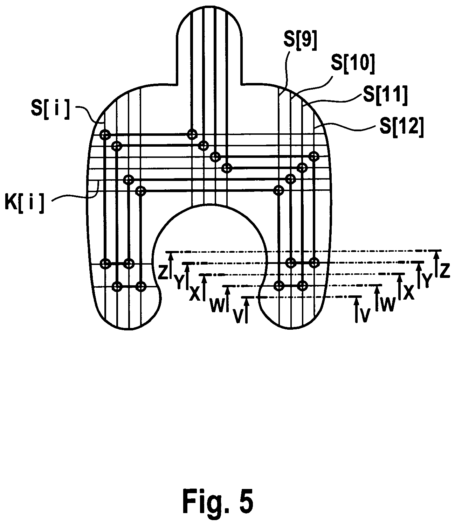

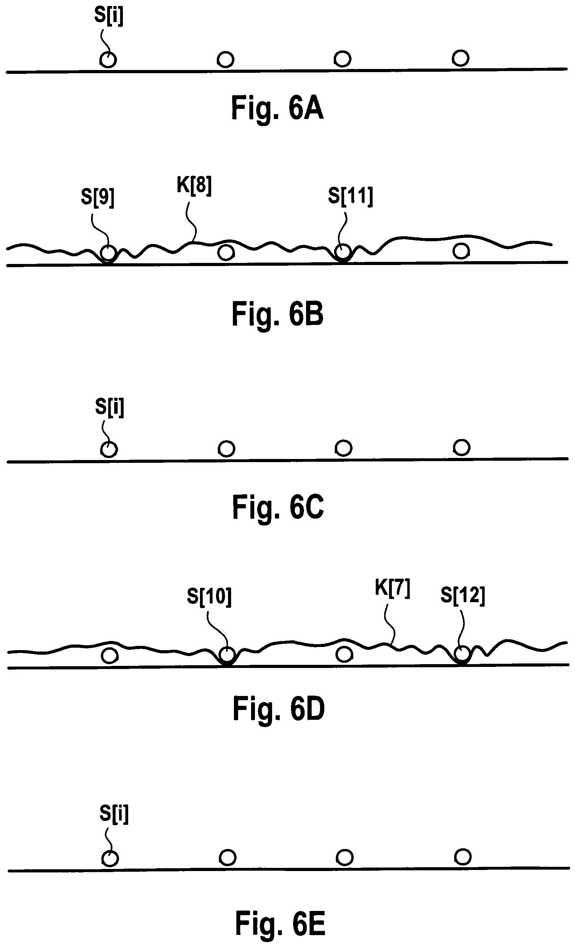

Die Fig. 6A bis 6E zeigen die Verknüpfungen der Kett- und Schussfäden des Pads in den Schnittebenen, die in Fig. 5 dargestellt sind. In der Schnittebene V - V sind die Kettfäden K [i] und Schussfäden S [i] nicht verknüpft, da Kettfäden in dieser Ebene nicht vorhanden sind. In der Schnittebene W - W ist der Kettfaden K [8] mit dem Schussfaden S [9] sowie der Kettfaden K[8] mit dem Schussfaden S [11] verknüpft, so dass eine elektrische Verbindung zwischen Kett- und Schussfaden hergestellt wird. In der Schnittebene X - X sind die Kettfäden K [i] und Schussfäden S [i] nicht verknüpft, da Kettfäden in dieser Ebene nicht vorhanden sind. In der Schnittebene Y - Y ist der Kettfaden K [7] mit dem Schussfaden S [10] sowie der Kettfaden K [7] mit dem Schussfaden S [12] verknüpft, so dass eine elektrische Verbindung zwischen Kett- und Schussfaden hergestellt wird. In der Schnittebene Z - Z sind die Kettfäden K [i] und Schussfäden S [i] nicht verknüpft, da Kettfäden in dieser Ebene nicht vorhanden sind.

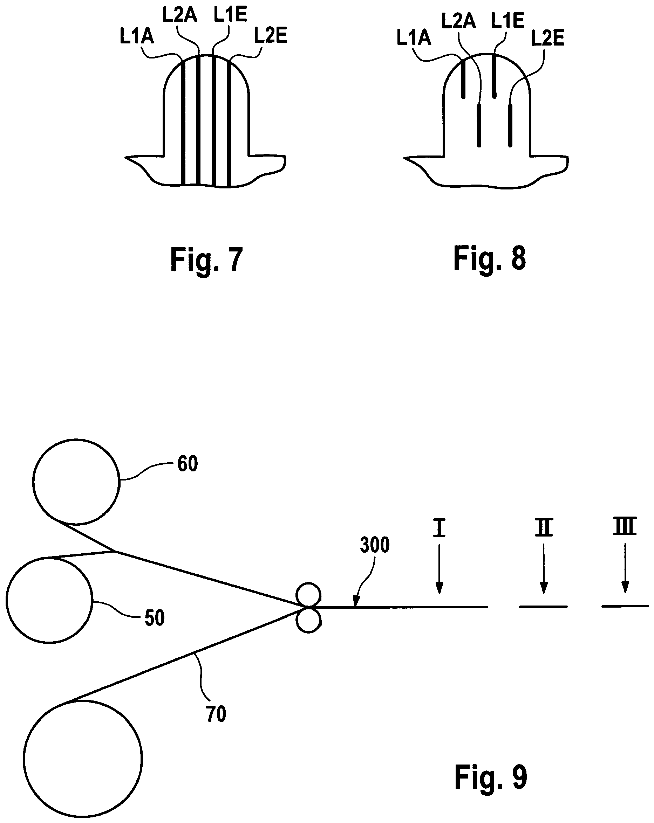

Fig. 7 zeigt in schematischer Darstellung die Anordnung der Anschlusskontakte L1A, L1E und L2A, L2E an der Lasche 200E der oben beschriebenen Ausführungsformen des Pads. Die Enden der Kett- oder Schussfäden verlaufen bei diesen Ausführungsformen in gleichem Abstand bis an den Rand der Lasche. Zur Bildung der Anschlusskontakte L1A, L1E und L2A, L2E befinden sich die Fäden an der Oberfläche der Lasche 200E. Um einen Kurzschluss zwischen den Anschlusskontakten des Anschlussteils zu vermeiden, muss die Breite oder der Durchmesser der Anschusskontakte des Anschlussteils kleiner als der Abstand zwischen den Anschlusskontakten L1A und L2A, L2A und L1E sowie L1E und L2E des Pads sein. Damit ist die Breite bzw. der Durchmesser der Anschlusskontakte des Anschlussteils beschränkt.

Fig. 8 zeigt eine alternative Ausführungsform der Anordnung der Anschlusskontakte an der Lasche 200E des Pads in schematischer Darstellung, bei der die Anschlusskontakte L1A und L1E der einen Leiterbahn L1A-L1E zu den Anschlusskontakten L2A und L2E der anderen Leiterbahn L2A-L2E versetzt angeordnet sind. Die Anschlusskontakte L1A und L1E der einen Leiterbahn befinden sich dabei auf der oberen Hälfte und die Anschlusskontakte L2A und L2E der anderen Leiterbahn auf der unteren Hälfte der Lasche 200E. Da das Pad an der Oberfläche eine isolierende gewebte Deckschicht aufweist, ist ein gezieltes "Abtauchen" der Fäden möglich. Bei der Ausführungsform von Fig. 8 liegen die Schussfäden S [5] und S [7] (Fig. 3) in der in Fig. 8 oberen Hälfte der Lasche unterhalb der Deckschicht und die Schussfäden S [6] und S [8] (Fig. 3) in der unteren Hälfte der Lasche unterhalb der Deckschicht, so dass die Anschlusskontakte des Anschlussteils eine größere Breite oder einen größeren Durchmesser als bei dem Ausführungsbeispiel von Fig. 7 haben können, ohne dass ein Kurzschluss zwischen den Kontakten L1A und L1E bzw. L2A und L2E entsteht.

Nachfolgend wird das erfindungsgemäße Webverfahren zur Herstellung der Feuchtigkeitssensoren im Einzelnen beschrieben.

Fig. 9 zeigt die wesentlichen Verfahrensschritte zur Herstellung der Feuchtigkeitssensoren in stark vereinfachter schematischer Darstellung. Zur Herstellung der vorzugsweise mehrlagigen Gewebebahn 200 werden die Kettfäden 50 und die Schussfäden 60 zugeführt. Nach der Herstellung des Gewebes erfolgen weitere dem Fachmann bekannte Verfahrensschritte, die aber nicht beschrieben werden. Dazu zählt beispielsweise das Veredeln, insbesondere das Waschen, das Fixieren oder eine Wärmebehandlung.

Während des Webprozesses wird eine Lage 70 zugeführt, mit der die Gewebebahn kaschiert wird. Die Lage 70 wird auf die Unterseite der Gewebebahn 80 aufgebracht. Dann werden in einem weiteren Verfahrensschritt I die einzelnen Feuchtigkeitssensoren separiert. In einem weiteren Prozessschritt II werden die Feuchtigkeitssensoren überprüft. Schließlich werden die Sensoren konfektioniert III.

Neben den elektrischen Eigenschaften ist das Benetzungsverhalten für die Funktion der Feuchtigkeitssensoren ausschlaggebend. In Versuchen hat sich gezeigt, dass sich mit Spinnpräparationen (Avivage) das Benetzungsverhalten der Feuchtigkeitssensoren positiv beeinflussen lässt. Ohne Spinnpräparationen zeigen die Feuchtigkeitssensoren ein hydrophobes Verhalten. Daher werden die Fäden 50, 60 bei dem erfindungsgemäßen Verfahren mit einer Spinnpräparation ausgerüstet, sodass sich ein hydrophiles Verhalten zeigt.

Durch das unterschiedliche Anheben bzw. Absenken der Kettfäden, das unterschiedliche Bindungsarten im Gewebe zur Folge hat, entstehen pro Längeneinheit unterschiedliche Kettfadenlängen. Die Kettfäden (x Kettfäden) mit einer Länge (y m) werden auf einem Kettbaum aufgewickelt, bevor die Kettfäden in der Webmaschine verarbeitet werden. Durch die unterschiedlichen Längen im Gewebe entstehen Spannungen in den Kettfäden bzw. im Gewebe. Um diese Spannungen ausgleichen zu können, werden nach einer bestimmten Wegstrecke, d. h. nach dem Weben einer oder mehrerer Zeilen mit nebeneinander angeordneten Feuchtigkeitssensoren, Ausgleichstreifen 350 in das Gewebe eingewebt. Die Breite der Ausgleichstreifen 350 und der Abstand aufeinanderfolgender Ausgleichstreifen werden so dimensioniert, dass die in den Kettfäden vorhandenen Spannungen aufgehoben werden.

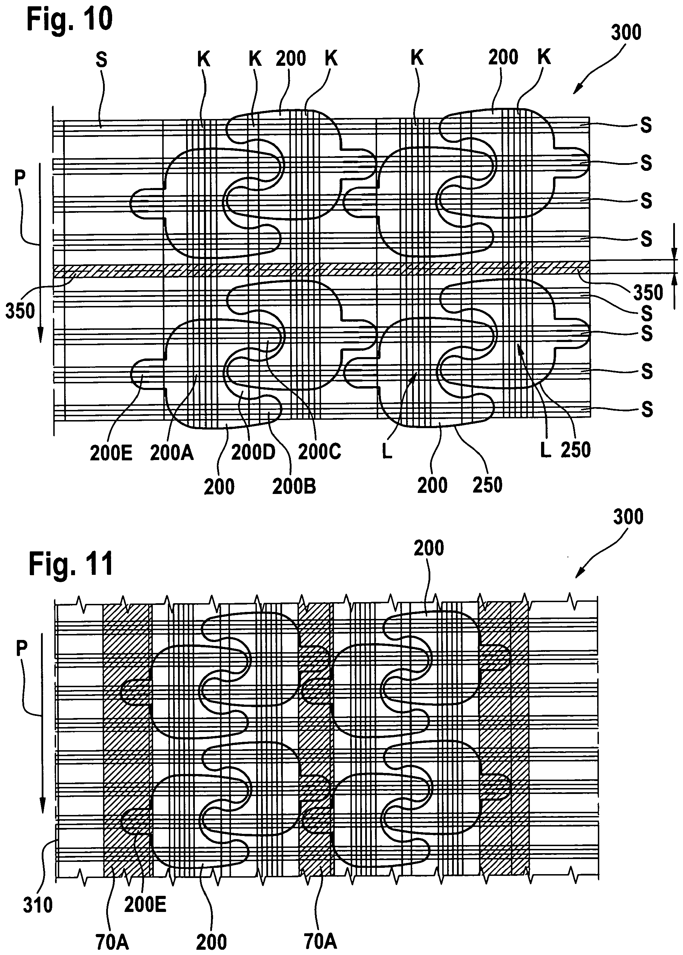

Fig. 10 zeigt einen Abschnitt der Gewebebahn 300 zur Herstellung der Feuchtigkeitssensoren 200. Die Produktionsrichtung P der Gewebebahn ist mit einem Pfeil gekennzeichnet. Die Schussfäden S und Kettfäden K zur Herstellung der Feuchtigkeitssensoren 200 sind wieder durch Linien dargestellt. Die Schussfäden S verlaufen quer zur Produktionsrichtung, während die Kettfäden K zur Produktionsrichtung verlaufen. Schuss- und Kettfäden S, K bilden eine Art Gitternetz, in das sich die Konturen der Feuchtigkeitssensoren einfügen. Fig. 10 zeigt, dass die Ausgleichsstreifen 350 zwischen den Feuchtigkeitssensoren 200 über die gesamte Breite der Gewebebahn quer zur Produktionsrichtung verlaufen.

Nachfolgend wird die Anordnung der Feuchtigkeitssensoren sowie der elektrisch leitfähigen Kett- und Schussfäden im Einzelnen beschrieben.

Auf der Gewebebahn 300 werden quer zur Produktionsrichtung P, d. h. über die gesamte Breite der Gewebebahn, mehrere Feuchtigkeitssensoren 200 nebeneinander gewebt. Fig. 10 zeigt einen Nutzen der Gewebebahn. In der Praxis hat die Gewebebahn eine Breite von 1000 mm, wobei die Gewebebahn in drei Nutzen von jeweils einer Breite von 306 mm unterteilt wird. Auf einem Nutzen sind vier Feuchtigkeitssensoren nebeneinander angeordnet. Daraus ergibt sich, dass in einer Reihe zwölf Sensoren angeordnet sind.

Bei dem vorliegenden Ausführungsbeispiel werden quer zur Produktionsrichtung jeweils vier Feuchtigkeitssensoren nebeneinander gewebt, wobei die Feuchtigkeitssensoren verschachtelt zueinander angeordnet werden. Es bilden also insgesamt acht verschachtelte Feuchtigkeitssensoren eine vorausgehende Gruppe von Sensoren, die quer zur Produktionsrichtung nebeneinander gewebt werden, und acht verschachtelte Feuchtigkeitssensoren eine nachfolgende Gruppe von Feuchtigkeitssensoren, die quer zur Produktionsrichtung nebeneinander gewebt werden. Zwischen den beiden Gruppen von Sensoren verläuft der Ausgleichsstreifen 350.

Die einzelnen Feuchtigkeitssensoren einer Gruppe von Feuchtigkeitssensoren sind derart versetzt zueinander angeordnet, dass jeweils ein Schenkel 200B, 200C eines Feuchtigkeitssensors 200 in einen Ausschnitt 200D eines anderen Feuchtigkeitssensors greift. Damit liegen die Feuchtigkeitssensoren 200 dicht nebeneinander, ohne sich dabei zu berühren.

Fig. 10 zeigt, dass Konturen der Feuchtigkeitssensoren auf das Gitternetz der Fäden S, K ausgerichtet sind oder umgekehrt. Die quer zur Produktionsrichtung verlaufenden Schussfäden S bilden jeweils in einem gleichen Abstand aufeinanderfolgende Gruppen von Schussfäden, die jeweils vier in gleichem Abstand zueinander angeordnete Schussfäden umfassen. Die beiden äußeren Gruppen von Schussfäden S verlaufen durch den mittleren Bereich einer der beiden Schenkel 200B, 200C des einen Feuchtigkeitssensors 200 bzw. durch die Lasche 200E und den mittleren Bereich 200A sowie den Ausschnitt 200D des anderen Feuchtigkeitssensors, der mit dem einen Sensor verschachtelt ist, während die mittleren Gruppen von Schussfäden S durch die Lasche 200E und den mittleren Bereich 200A sowie den Ausschnitt 200D des einen Feuchtigkeitssensors 200 bzw. den mittleren Bereich einer der beiden Schenkel 200B, 200C des anderen Feuchtigkeitssensors verlaufen.

Die in Produktionsrichtung P verlaufenden Kettfäden K bilden zwei Gruppen, die die gleiche Anzahl von Kettfäden umfassen sowie eine Gruppe, die eine kleinere Anzahl von Kettfäden umfasst. Die Gruppen mit der größeren Anzahl von Kettfäden erstrecken sich durch den mittleren Bereich 200A der Feuchtigkeitssensoren 200, während sich die Gruppen mit der kleineren Anzahl von Kettfäden durch die Schenkel 200B, 200C der Feuchtigkeitssensoren erstrecken. Dabei verlaufen die Kett- und Schussfäden jeweils orthogonal zueinander, sodass sich Kreuzungspunkte ergeben, an denen die Kontaktierung der Kett- und Schussfäden erfolgen kann (Fig. 3).
Fig. 10 zeigt, dass sich mit einer weitgehend symmetrischen Aufteilung von quer zueinander verlaufenden Kett- und Schussfäden ohne Unterbrechungen die Leiterbahnstrukturen L auf den Feuchtigkeitssensoren 200, die eine vorgegebene Kontur 250 haben, schaffen lassen, wobei sich die Feuchtigkeitssensoren ohne Zerstörung der elektrisch leitenden Strukturen wieder voneinander trennen lassen. Mit dieser versetzten oder verschachtelten Anordnung können die Produktionskosten gesenkt werden.

Nachfolgend wird das Aufbringen der Lage 70 auf die Gewebebahn 300 unter Bezugnahme auf Fig. 11 im Einzelnen beschrieben. Die Gewebebahn 300 weist eine dem Patienten zugewandte Rückseite und eine dem Patienten abgewandte Vorderseite auf. Bei der Lage 70 handelt es sich um eine Klebefolie, die mit einer Abziehfolie (Liner) versehen ist. Die Klebefolie weist ebenfalls eine dem Patienten zugewandte Rückseite und eine dem Patienten abgewandte Vorderseite auf. Die Klebefolie ist an der Vorder- und Rückseite mit einer Klebeschicht versehen. Die Vorderseite der Klebefolie wird mit der Rückseite der Gewebebahn verklebt (Fig. 9). Die Klebeschicht auf der Rückseite der doppelseitigen Klebefolie wird von der Abziehfolie geschützt, die vor dem Aufbringen des Feuchtigkeitssensors auf die Haut des Patienten abgezogen und verworfen wird. Die obere Klebeschicht und die untere Klebeschicht der Klebefolie können unterschiedliche Eigenschaften haben. Die obere Klebeschicht ist dafür bestimmt, Gewebebahn und Klebefolie fest miteinander zu verbinden, während die untere Klebeschicht nur der Adhäsion des Sensors auf der Haut des Patienten dient. Nachfolgend wird die mit der Abziehfolie versehene Klebefolie (Lage) der einfachhalthalber auch als Klebefolie 70 bezeichnet.

Die Klebeschicht an der Unterseite der Klebefolie 70 weist Bereiche auf, die frei von Klebestoff sind. Diese Bereiche sind in Produktionsrichtung P verlaufende Zonen 70A, die in Fig. 11 schraffiert sind. Diese klebefreien Zonen 70A sollen beim Kaschieren von Gewebebahn 300 und Klebefolie 70 unterhalb der Anschlusslaschen 200E der Feuchtigkeitssensoren 200 zu liegen kommen,, da der Feuchtigkeitssensor im Bereich der Lasche 200E nicht auf der Haut des Patienten haften soll, ansonsten aber auf der Haut des Patienten vollflächig haften soll. Hierfür ist eine exakte Ausrichtung der klebefreien Zonen und der Laschen der Sensoren erforderlich.
Zur exakten Ausrichtung von klebefreien Zonen 70A und Anschlusslaschen 200E dienen in Produktionsrichtung P verlaufende Markierungselemente 310, bei denen es sich um leitfähige Kettfäden K handeln kann, die an die Oberfläche des Gewebes gelegt werden, sodass sich die Kettfäden von dem Gewebe optisch abheben. Dadurch wird eine Nulllinie 310 geschaffen. Alternativ können die Markierungselemente auch mit kontrastreichen Fäden gebildet werden, die nicht leitfähig sind.

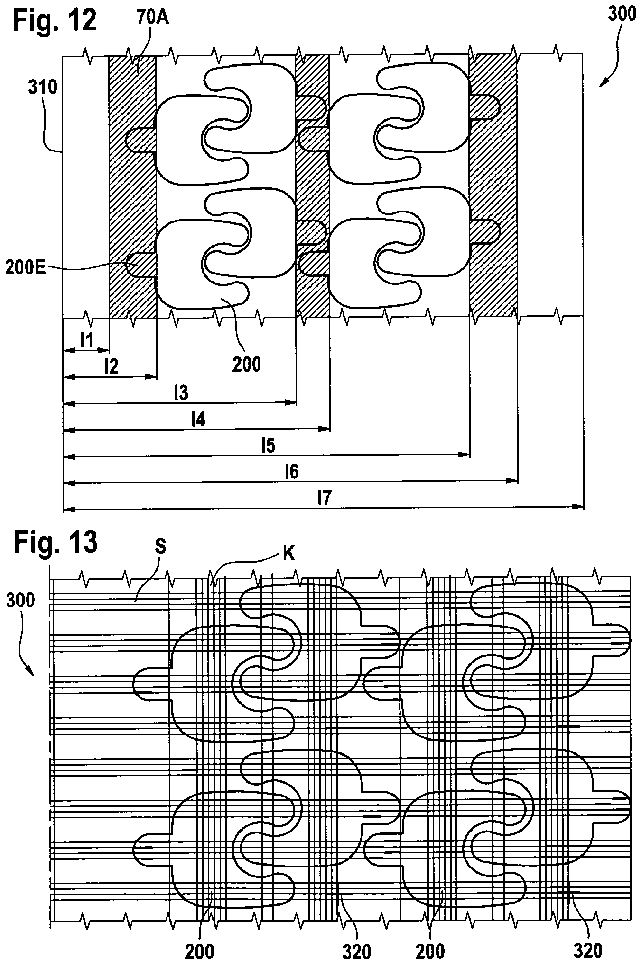

Fig. 12 zeigt die Vermaßung der klebefreien Zone 70A, die deckungsgleich unter den Laschen 200E liegen, mithilfe der Nulllinie 310, die von dem kontrastreichen Kettfaden K gebildet wird.

Die Separierung der einzelnen Feuchtigkeitssensoren 200 erfolgt bei dem erfindungsgemäßen Verfahren mit einem Laser. Der Vorteil eines Lasers liegt darin, dass beim Ausschneiden der Feuchtigkeitssensoren das Ausfransen der Fäden vermieden wird, da die Kanten durch die thermische Einwirkung des Lasers versiegelt werden. Weitere Vorteile liegen in der besonders hohen Genauigkeit, die sich aus der präzisen Führung des Laserstrahls und dessen kleinen Durchmesser ergibt. Auch lassen sich mit dem Laser unterschiedliche Geometrien einfach schneiden. Die Sensoren können alternativ auch durch Ultraschallstanzen separiert werden. Das Ultraschallstanzen kann beispielsweise mit einer Frequenz von 40 kHz erfolgen.

Die exakte Führung des Lasers oder der Ultraschallstanze setzt eine genaue Bestimmung der Lage der Kontur 250 des Feuchtigkeitssensors 200 voraus. Zur Positionsbestimmung sind bei dem erfindungsgemäßen Verfahren kreuzförmige Markierungselemente 320 vorgesehen, die zwischen den Feuchtigkeitssensoren 200 gewebt werden. Die kreuzförmigen Markierungselemente 320 werden durch leitfähige Kett- und Schussfäden K, S gebildet, die sich kreuzen. Diese leitfähigen Fäden werden an die Oberfläche der Gewebebahn 300 gelegt, so dass sie sich von einem automatisierten Bildverarbeitungssystem erfassen lassen. Alternativ können die Markierungselemente wieder durch nicht-leitfähige, aber kontrastreiche Fäden gebildet werden.

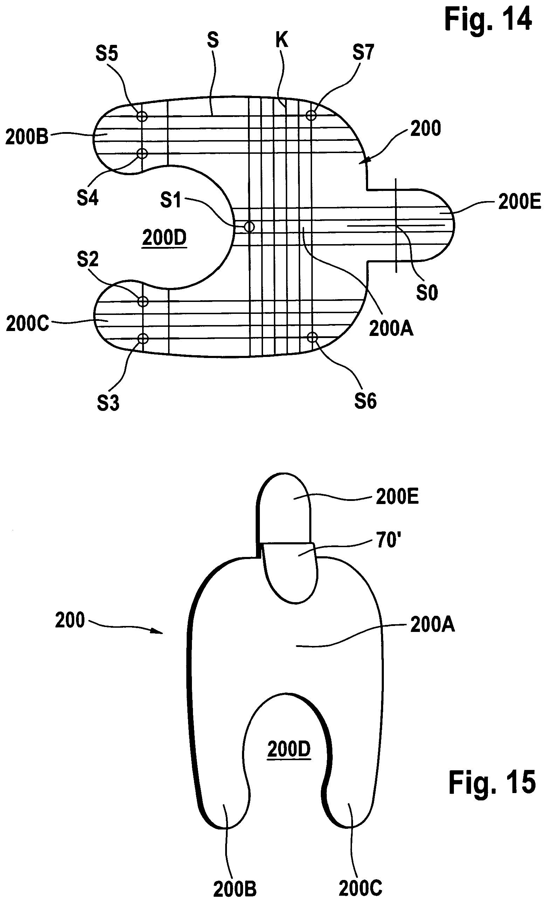

Fig. 13 zeigt die Anordnung der kreuzförmigen Markierungselemente 320 auf der Gewebebahn 300 außerhalb der Feuchtigkeitssensoren 200. Die kreuzförmigen Markierungselemente 320, mit denen die Position der Feuchtigkeitssensoren gekennzeichnet wird, werden von einem Kamerasystem erfasst und mit einem Bildverarbeitungssystem verarbeitet. Diese Einrichtungen sind dem Fachmann bekannt. Nach dem Separieren der Feuchtigkeitssensoren mittels des Lasers erfolgt eine Kontrolle der Feuchtigkeitssensoren innerhalb des Prozesses (In-Prozess-Kontrolle (IPK)). Dabei wird überprüft, ob vorgegebene Kontrollpunkte auf dem Feuchtigkeitssensor 200 innerhalb der Schneidkontur liegen. Bei den vorgegebenen Kontrollpunkten handelt es sich um die Kreuzungspunkte von sich kreuzenden Kett- und Schussfäden K, S. Diese Kontrollpunkte sind in Fig. 14 mit S1 bis S7 bezeichnet. Mit einem Kamerasystem werden die Schnittpunkte S1 - S7 beim Ausschneiden der Sensoren erfasst und mit dem Bildverarbeitungssystem wird überprüft, ob die Schnittpunkte bezogen auf einen vorgegebenen Nullpunkt S0, der in der Lasche 200E des Feuchtigkeitssensors 200 liegt, innerhalb eines definierten Toleranzfeldes liegen. In dem Bildverarbeitungssystem können Minimal- und Maximalwerte für die Koordinaten der Schnittpunkte S hinterlegt sein. Mit dem Bildverarbeitungssystem werden die Abweichungen der Ist-Werte von den SollWerten berechnet, wobei auf einen fehlerhaften Sensor geschlossen wird, wenn die Abweichungen vorgegebene Grenzwerte überschreiten. In diesem Fall werden die entsprechenden Feuchtigkeitssensoren aussortiert. Die In-Prozess-Kontrolle kann auch die Überprüfung der elektrischen Eigenschaften der Leiterbahnen einschließen. Hierzu wird überprüft, ob der elektrische Widerstand zwischen den Leiterbahnen L1A/L1E innerhalb eines vorgegebenen Soll-Wertbereichs und der Widerstand zwischen den Leiterbahnen L2A/L2E innerhalb eines vorgegebenen Soll-Wertbereichs liegt. Darüber hinaus wird überprüft, ob zwischen den Leiterbahnen L1 A/L2A bzw. L1E/L2E kein Kurzschluss besteht.

Nach dem Separieren und Überprüfen werden die Feuchtigkeitssensoren konfektioniert. Da die Abziehfolie (Liner) im Bereich der Lasche nicht mit der Klebefolie verklebt ist, da die Klebefolie an der Rückseite im Bereich der Lasche frei von Kleber ist, kann die Abziehfolie leicht im Bereich der Lasche gegriffen werden und von dem Feuchtigkeitssensor abgezogen werden. Der Abschnitt des Liners im Bereich der Lasche dient somit als Abziehhilfe. Um diesen Abschnitt leicht greifen zu können, wird der Liner im Bereich der Lasche um 180° gefalzt.

Fig. 15 zeigt eine Rückansicht des Feuchtigkeitsensors 200 mit der Lasche 200E und der im Bereich der Lasche 200E gefalzten Abziehfolie (Liner) 70'. Die Linie, an der die Abziehfolie 70' gefalzt wird, läuft parallel zu der Linie, an der die Lasche 200E an den mittleren Bereich 200A des Feuchtigkeitssensors 200 anschließt, d.h. parallel zu der Linie, die den klebefreien Bereich von dem mit Kleber versehenen Bereich der Klebefolie 70 trennt (Fig. 11). Die Biegekante des gefalzten Linerabschnitts erstreckt sich, beispielsweise um 2 mm, in die Lasche und ermöglicht so das Changieren der klebefreien Kante aufgrund von Toleranzen. Die im Bereich der Lasche abstehende Abziehfolie (Liner) lässt sich leicht greifen, so dass sich der Liner von der Klebefolie leicht abziehen lässt. Da der Feuchtigkeitssensor im Bereich der Lasche wegen der dort fehlenden Klebeschicht nicht an der Haut des Patienten haftet, kann auch der Feuchtigkeitssensor nach dem Gebrauch an der Lasche leicht gegriffen und von der Haut abgezogen werden.

## Patentansprüche

1. Webverfahren zur Herstellung einer Vielzahl von Feuchtigkeitssensoren für eine Vorrichtung zur Überwachung eines Patientenzugangs, wobei zur Herstellung der Feuchtigkeitssensoren nicht-leitfähige Kettfäden und nicht-leitfähige Schussfäden sowie leitfähige Kettfäden und leitfähige Schussfäden in einem textilen Flächengebilde derart angeordnet werden, dass räumlich abgrenzbare Strukturen aus elektrischen Leiterbahnen geschaffen werden,
**dadurch gekennzeichnet, dass**
die Feuchtigkeitssensoren jeweils einen inneren Bereich mit zwei Schenkeln aufweisen, die einen zentralen Ausschnitt seitlich umschließen,
eine Mehrzahl von einzelnen Feuchtigkeitssensoren auf einer gemeinsamen Gewebebahn quer zur Produktionsrichtung nebeneinander gewebt werden und nach dem Weben die einzelnen Feuchtigkeitssensoren voneinander separiert werden und
die Feuchtigkeitssensoren einer Gruppe von Feuchtigkeitssensoren, die quer zur Produktionsrichtung nebeneinander gewebt werden, zueinander verschachtelt angeordnet werden, wobei die Feuchtigkeitssensoren auf der Gewebebahn derart zueinander versetzt angeordnet werden, dass jeweils ein Schenkel eines Feuchtigkeitssensors in einen Ausschnitt eines anderen Feuchtigkeitssensors greift.

2. Webverfahren nach Anspruch 1, **dadurch gekennzeichnet, dass** zwischen einer vorausgehenden Gruppe von Feuchtigkeitssensoren, die quer zur Produktionsrichtung nebeneinander gewebt werden, und einer nachfolgenden Gruppe von Feuchtigkeitssensoren, die quer zur Produktionsrichtung nebeneinander gewebt werden, ein quer zur Produktionsrichtung verlaufender Ausgleichsstreifen gewebt wird.

3. Webverfahren nach Anspruch 1 oder 2, **dadurch gekennzeichnet, dass** sich kreuzende leitfähige Kettfäden und Schussfäden zur Bildung der Leiterbalmstrukturen derart angeordnet werden, dass die Kett- und Schussfäden an einzelnen Kreuzungspunkten kontaktieren.

4. Webverfahren nach einem der Ansprüche 1 bis 3, **dadurch gekennzeichnet, dass** die Leiterbahnstrukturen der einzelnen Feuchtigkeitssensoren aus Gruppen von leitfähigen Kettfäden, die jeweils eine Mehrzahl von in einer ersten Richtung verlaufenden leitfähigen Kettfäden umfassen, und Gruppen von leitfähigen Schussfäden, die jeweils eine Mehrzahl von in einer zu der ersten Richtung orthogonalen Richtung nebeneinander verlaufenden leitfähigen Schussfäden umfassen, gebildet werden, wobei die Gruppen von Kettfäden im gleichen Abstand zueinander angeordnet sind und/oder die Gruppen von Schussfäden im gleichen Abstand zueinander angeordnet sind.

5. Webverfahren nach Anspruch 4, **dadurch gekennzeichnet, dass** die leitfähigen Kettfäden in Produktionsrichtung und die leitfähigen Schussfäden quer zur Produktionsrichtung verlaufen.

6. Webverfahren nach einem der Ansprüche 1 bis 5, **dadurch gekennzeichnet, dass** die Leiterbahnstrukturen der Feuchtigkeitssensoren jeweils von drei Gruppen von Schussfäden und zwei Gruppen von Kettfäden gebildet werden.

7. Webverfahren nach einem der Ansprüche 1 bis 6, **dadurch gekennzeichnet, dass** in Produktionsrichtung verlaufende Markierungselemente und/oder zwischen den Feuchtigkeitssensoren kreuzförmige Markierungselemente gewebt werden, wobei die Markierungselemente durch leitfähige Kett- und/oder Schussfäden gebildet werden, die an die Oberfläche des Gewebes gelegt werden.

8. Webverfahren nach einem der Ansprüche 1 bis 7, **dadurch gekennzeichnet, dass** auf die der Haut des Patienten zugewandte Rückseite der Gewebebahn eine doppelseitig klebende Klebefolie aufgebracht wird, die an Rückseite, die auf der Haut des Patienten zu liegen kommt, mit einer abziehbaren Abziehfolie versehen ist, die die Klebeschicht an der Rückseite der Klebefolie schützt, wobei die Klebefolie an der Rückseite, die zum Aufkleben auf die Haut des Patienten bestimmt ist, in vorgegebenen Abständen Abschnitte aufweist, in denen die Klebefolie frei von Kleber ist.

9. Webverfahren nach einem der Ansprüche 1 bis 8, **dadurch gekennzeichnet, dass** die Feuchtigkeitssensoren jeweils Laschen aufweisen, an der die Leiterbahnen Anschlusskontakte bilden.

10. Webverfahren nach einem der Ansprüche 1 bis 9, **dadurch gekennzeichnet, dass** die einzelnen Feuchtigkeitssensoren mittels eines Lasers voneinander separiert werden.

11. Webverfahren nach einem der Ansprüche 1 bis 10, **dadurch gekennzeichnet, dass** nach dem Separieren der einzelnen Feuchtigkeitssensoren die Position von definierten Kontrollpunkten auf dem Feuchtigkeitssensor mittels eines Bildverarbeitungssystems überprüft wird, wobei auf einen fehlerhaften Feuchtigkeitssensor geschlossen wird, wenn die Kontrollpunkte nicht innerhalb eines vorgegebenen Toleranzfeldes liegen.

12. Webverfahren nach Anspruch 11, **dadurch gekennzeichnet, dass** die Kontrollpunkte die Kreuzungspunkte von sich kreuzenden leitfähige Kett- und Schussfäden sind.

13. Vorrichtung zum Weben, **dadurch gekennzeichnet, dass** die Vorrichtung derart konfiguriert ist, dass das Webverfahren nach einem der Ansprüche 1 bis 12 durchführbar ist.

## Claims

1. A weaving method for producing a plurality of moisture sensors for a device for monitoring a patient access,
in order to produce the moisture sensors, non-conductive warp threads and non-conductive weft threads as well as conductive warp threads and conductive weft threads being disposed in a textile two-dimensionally extending structure, in such a way that spatially demarcatable structures of electrical strip conductors are created,
characterised that,
the moisture sensors each comprise a central region with two legs which laterally surround a central cutout,
a plurality of individual moisture sensors are woven beside one another normal to the production direction on a common woven fabric web and, after the weaving, the individual moisture sensors are separated from one another, and
the moisture sensors of a group of moisture sensors, which are woven beside one another normal to the production direction, are disposed interlaced with respect to one another, the moisture sensors on the woven fabric web being disposed offset with respect to one another, in such a way that in each case a leg of a moisture sensor engages in a cutout of another moisture sensor.

2. The weaving method according to claim 1, **characterised in that** a compensation strip running normal to the production direction is woven between a preceding group of moisture sensors, which are woven beside one another normal to the production direction, and a subsequent group of moisture sensors, which are woven beside one another normal to the production direction.

3. The weaving method according to claim 1 or 2, **characterised in that** intersecting conductive warp threads and weft threads are disposed for the formation of the strip conductor structures in such a way that the warp and weft threads make contact at individual points of intersection.

4. The weaving method according to any one of claims 1 to 3, **characterised in that** the strip conductor structures of the individual moisture sensors are formed from groups of conductive warp threads, which each comprise a plurality of conductive warp threads running in a first direction, and groups of conductive weft threads, which each comprise a plurality of conductive weft threads running beside one another in a direction at right angles to the first direction, wherein the groups of warp threads are disposed with an equal spacing from one another and/or the groups of weft threads are disposed with an equal spacing from one another.

5. The weaving method according to claim 4, **characterised in that** the conductive warp threads run in the production direction and the conductive weft threads run normal to the production direction.

6. The weaving method according to any one of claims 1 to 5, **characterised in that** the strip conductor structures of the moisture sensors are each formed by three groups of weft threads and two groups of warp threads.

7. The weaving method according to any one of claims 1 to 6, **characterised in that** marking elements running in the production direction and/or cross-shaped marking elements between the moisture sensors are woven, the marking elements being formed by conductive warp and/or weft threads which are laid at the surface of the woven fabric.

8. The weaving method according to any one of claims 1 to 7, **characterised in that** there is applied on the rear side of the woven fabric web facing the patient's skin a two-sided adhesive film, which is provided with a tear-off film that can be torn off at the rear side that lies on the patient's skin, said tear-off film protecting the adhesive layer at the rear side of the adhesive film, wherein the adhesive film comprises at predetermined intervals at the rear side, which is to be stuck onto the patient's skin, sections in which the adhesive film is free from adhesive.

9. The weaving method according to any one of claims 1 to 8, **characterised in that** the moisture sensors each comprise tabs on which the strip conductors form terminal contacts.

10. The weaving method according to any one of claims 1 to 9, **characterised in that** the individual moisture sensors are separated from one another by means of a laser.

11. The weaving method according to any one of claims 1 to 10, **characterised in that**, after the separation of the individual moisture sensors, the position of defined control points on the moisture sensor is checked by means of an image processing system, wherein it is concluded that there is a faulty moisture sensor when the control points do not lie within a predetermined tolerance field.

12. The weaving method according to claim 11, **characterised in that** the control points are the points of intersection of intersecting conductive warp and weft threads.

13. A device for weaving, **characterised in that** the device is configured in such a way that the weaving method can be implemented according to any one of claims 1 to 12.

## Revendications

1. Procédé de tissage pour fabriquer une pluralité de capteurs d'humidité pour un dispositif de surveillance d'un accès au patient, dans lequel pour fabriquer les capteurs d'humidité, des fils de chaîne non conducteurs et des fils de trame non conducteurs ainsi que des fils de chaîne conducteurs et des fils de trame conducteurs sont disposés en une structure plate textile de telle manière que des structures pouvant être délimitées spatialement sont créées à partir de pistes conductrices électriques,
**caractérisé en ce que**
les capteurs d'humidité présentent respectivement une zone intérieure avec deux branches qui renferment latéralement une partie centrale,
une pluralité de divers capteurs d'humidité sont tissés côte à côte sur une bande de tissu commune de manière transversale par rapport au sens de production et les divers capteurs d'humidité sont séparés les uns des autres après le tissage et les capteurs d'humidité d'un groupe de capteurs d'humidité, qui sont tissés côte à côte de manière transversale par rapport au sens de production, sont disposés de manière imbriquée les uns par rapport aux autres, les capteurs d'humidité étant disposés de manière décalée les uns par rapport aux autres sur la bande de tissu de telle manière que respectivement une branche d'un capteur d'humidité s'engage dans une partie d'un autre capteur d'humidité.

2. Procédé de tissage selon la revendication 1, **caractérisé en ce qu'**un ruban de compensation s'étendant de manière transversale par rapport au sens de production est tissé entre un groupe, qui précède, de capteurs d'humidité, qui sont tissés côte à côte de manière transversale par rapport au sens de production, et un groupe, qui suit, de capteurs d'humidité, qui sont tissés côte à côte de manière transversale par rapport au sens de production.

3. Procédé de tissage selon la revendication 1 ou 2, **caractérisé en ce que** des fils de chaîne et des fils de trame conducteurs se croisant sont disposés pour former les structures de piste conductrice de telle manière que les fils de chaîne et les fils de trame sont mis en contact sur divers points de croisement.

4. Procédé de tissage selon l'une quelconque des revendications 1 à 3, **caractérisé en ce que** les structures de piste conductrice des divers capteurs d'humidité sont formées à partir de groupes de fils de chaîne conducteurs, qui comprennent respectivement une multitude de fils de chaîne conducteurs s'étendant dans une première direction, et de groupes de fils de trame conducteurs, qui comprennent respectivement une multitude de fils de trame conducteurs s'étendant côte à côte dans une direction orthogonale par rapport à la première direction, dans lequel les groupes de fils de chaîne sont disposés à la même distance les uns par rapport aux autres et/ou les groupes de fils de trame sont disposés à la même distance les uns par rapport aux autres.

5. Procédé de tissage selon la revendication 4, **caractérisé en ce que** les fils de chaîne conducteurs s'étendent dans le sens de production et les fils de trame conducteurs s'étendent de manière transversale par rapport au sens de production.

6. Procédé de tissage selon l'une quelconque des revendications 1 à 5, **caractérisé en ce que** les structures de piste conductrice des capteurs d'humidité sont formées respectivement par trois groupes de fils de trame et deux groupes de fils de chaîne.

7. Procédé de tissage selon l'une quelconque des revendications 1 à 6, **caractérisé par** le tissage d'éléments de marquage s'étendant dans le sens de production et/ou d'éléments de marquage en forme de croix entre les capteurs d'humidité, les éléments de marquage étant formés par des fils de chaîne et/ou des fils de trame conducteurs qui sont placés sur la surface du tissu.

8. Procédé de tissage selon l'une quelconque des revendications 1 à 7, **caractérisé en ce qu'**est appliqué, sur le côté arrière, tourné vers la peau du patient, de la bande de tissu, un film adhésif collant sur deux côtés, qui est pourvu, sur le côté arrière qui vient se placer sur la peau du patient, d'un film détachable pouvant être détaché, qui protège la couche adhésive sur le côté arrière du film adhésif, dans lequel le film adhésif présente, sur le côté arrière qui se destine à être collé sur la peau du patient, à des distances prédéfinies, des sections dans lesquelles le film adhésif est sans colle.

9. Procédé de tissage selon l'une quelconque des revendications 1 à 8, **caractérisé en ce que** les capteurs d'humidité présentent respectivement des brides sur lesquelles les pistes conductrices forment des contacts de raccordement.

10. Procédé de tissage selon l'une quelconque des revendications 1 à 9, **caractérisé en ce que** les divers capteurs d'humidité sont séparés les uns des autres au moyen d'un laser.

11. Procédé de tissage selon l'une quelconque des revendications 1 à 10, **caractérisé en ce qu'**après la séparation des divers capteurs d'humidité, la position de points de contrôle définis est vérifiée sur le capteur d'humidité au moyen d'un système de traitement d'images, ce qui permet de conclure à la défaillance d'un capteur d'humidité quand les points de contrôle ne se situent pas à l'intérieur d'un champ de tolérances prédéfini.

12. Procédé de tissage selon la revendication 11, **caractérisé en ce que** les points de contrôle sont les points de croisement de fils de chaîne et de fils de trame conducteurs se croisant.

13. Dispositif de tissage, **caractérisé en ce que** le dispositif est configuré de telle manière que le procédé de tissage selon l'une quelconque des revendications 1 à 12 peut être mis en œuvre.
